# EUROPÄISCHE PATENTSCHRIFT

(11) **EP 3 259 061 B1**
(45) Veröffentlichungstag und Bekanntmachung des Hinweises auf die Patenterteilung: **10.04.2019**
(21) Anmeldenummer: 16706307.2
(22) Anmeldetag: 16.02.2016
(51) Int. Cl.: B01L 3/00, B01L 7/00, C12Q 1/6825

(54) **MIKROFLUIDISCHE VORRICHTUNG ZUR TEMPERATURGESTEUERTEN VERARBEITUNG EINER PROBENLÖSUNG**
MICROFLUIDIC DEVICE FOR THE TEMPERATURE-CONTROLLED PROCESSING OF A SAMPLE SOLUTION
DISPOSITIF MICROFLUIDIQUE DESTINÉ AU TRAITEMENT, COMMANDÉ PAR TEMPÉRATURE, D'UNE SOLUTION À EXAMINER

(30) Priorität: 20.02.2015 DE 102015002000
(43) Veröffentlichungstag der Anmeldung: 27.12.2017
(73) Patentinhaber: FRIZ Biochem Gesellschaft für Bioanalytik mbH, 82061 Neuried (DE)
(72) Erfinder: HARTWICH, Gerhard, 80639 München (DE); PERSIKE, Norbert, 81539 München (DE)
(74) Vertreter: Zeuner Summerer Stütz
(86) Internationale Anmeldenummer: PCT/EP2016/000257
(87) Internationale Veröffentlichungsnummer: WO 2016/131536

(56) Entgegenhaltungen:
- EP-A1- 3 000 529
- WO-A1-2010/060509
- WO-A2-2007/059167
- WO-A2-2008/064865
- US-A1- 2012 100 552
- WENMING WU ET AL: "Bubble-free on-chip continuous-flow polymerase chain reaction: concept and application", THE ANALYST, Bd. 136, Nr. 11, 1. Januar 2011 (2011-01-01), Seiten 2287-2293, XP055268745, GB ISSN: 0003-2654, DOI: 10.1039/c0an01034k

## Beschreibung

Die Erfindung betrifft eine Anordnung mit einer mikrofluidischen Vorrichtung zur temperaturgesteuerten Verarbeitung einer Probenlösung, mit einer Reaktionskammer, in der die Probenlösung auf eine Solltemperatur nahe des Normalsiedepunkts heizbar ist. Die Erfindung betrifft auch ein zugehöriges Verarbeitungsverfahren.

Zahlreiche Fragestellungen der molekularbiologischen Forschung und Diagnostik erfordern die Bestimmung der Menge bzw. Konzentration bestimmter Biomoleküle, beispielsweise bestimmter Nukleinsäuren in einer Probe. Der Begriff Nukleinsäure umfasst dabei im Rahmen dieser Beschreibung auch Nukleinsäuresequenzen.

Leistungsfähige und schnelle Detektionsverfahren setzen dabei eine Array-Technologie unter Verwendung sogenannter DNA-Chips ein, die eine oberflächensensitive Detektion von Nukleinsäureoligomer-Hybridisierungsereignissen ermöglichen. Oft wird der eigentlichen, ohnehin hochempfindlichen Detektion noch ein Amplifizierungsschritt vorgeschaltet, in dem die nachzuweisenden Nukleinsäuren vervielfältigt werden, damit sie letztlich in einer über der Nachweisgrenze der gewählten Detektionsmethode liegenden Konzentration vorhanden sind.

Für eine solche spezifische Amplifikation von Nukleinsäuren sind im Stand der Technik verschiedene Verfahren bekannt, beispielsweise die Polymerase-Kettenreaktion (PCR). Die PCR hat in fast allen Bereichen der Wissenschaft und Medizin, einschließlich der forensischen Medizin, der pränatalen Diagnostik, der Onkologie und nicht zuletzt in der mikrobiologischen Diagnostik Einzug gehalten. Bei der PCR handelt es sich um eine enzymatische Reaktion zur Amplifikation von Nukleinsäuren, die im Wesentlichen in einem wässrigen bzw. flüssigen Reaktionsgemisch mit sehr kleinen Volumina abläuft. Im Reaktionsgemisch befindet sich eine Nukleinsäure enthaltende Probe und die für die Reaktion außerdem notwendigen Primer, Nukleotide und eine Polymerase. Durch Zugabe von Puffern und vorzugsweise zweiwertigen Ionen wird das Reaktionsgemisch so eingestellt, dass die für die jeweilige Anwendungsart optimalen Reaktionsbedingungen herrschen.

Die PCR beruht auf einem sich mehrfach wiederholenden Zyklus aus drei, bei unterschiedlichen Temperaturen ablaufenden Schritten der Denaturierung, Hybridisierung und Verlängerung. In jedem Zyklus wird die Anzahl der zu vermehrenden Nukleinsäuren grob verdoppelt, so dass mit einer geringen Zahl an Zyklen eine signifikante Amplifikation erreicht werden kann.

Für weitere Details und Hintergründe zur PCR allgemein, zur Real Time PCR, bei der die Menge an Nukleinsäure bestimmt wird, während die PCR-Reaktion abläuft, und zu bevorzugten Verfahren zur elektrochemischen Detektion von Nukleinsäureoligomer-Hybridisierungsereignissen an Teststellen eines DNA-Chips wird auf die Druckschrift DE 10 2011 056 606 B3 der Anmelderin verwiesen.

Eine mikrofluidische Vorrichtung zur Verarbeitung von Biopartikeln ist beispielsweise aus der Druckschrift DE 10 2010 043 030 A1 und der US 2012/0100552 A1 bekannt.

In mikrofluidischen Vorrichtungen können Gasblasen nicht nur zu großen Temperaturunterschieden in der Probenlösung in der Reaktionskammer führen, sondern auch zu einem Herausdrücken von Probenlösung aus der Reaktionskammer und zu starken Flüssigkeitsoszillation im mikrofluidischen System. Um die Entstehung von Gasblasen in mikrofluidischen PCR-Systemen zu unterdrücken sind mehrere Maßnahmen vorgeschlagen worden, wie etwa eine besondere strukturelle Auslegung der Reaktionskammer, eine Oberflächenbehandlung der Reaktionskammerwände, eine Versiegelung der Reaktionskammer unter erhöhtem Druck, eine Entgasung der Probenlösung und der Zusatz von Reagenzien mit hohem Siedepunkt. Ausgehend davon liegt der Erfindung die Aufgabe zugrunde, eine mikrofluidische Vorrichtung der eingangs genannten Art anzugeben, bei der die Bildung unerwünschter Gasblasen in der Reaktionskammer unterdrückt und ein kontrollierter Ablauf der Verarbeitung in der Reaktionskammer gewährleistet ist.

Diese Aufgabe wird durch die Merkmale der unabhängigen Ansprüche gelöst. Weiterbildungen der Erfindung sind Gegenstand der abhängigen Ansprüche.

Gemäß der Erfindung ist bei einer gattungsgemäßen mikrofluidischen Vorrichtung vorgesehen, dass die Reaktionskammer über einen Ausgleichskanal mit einer im Normalbetrieb flüssigkeitsfreien Druckkammer in Fluidverbindung steht, welche mit einer zweiten Heizeinrichtung auf eine Temperatur oberhalb des Normalsiedepunkts der Probenlösung heizbar ist, und die ausgelegt ist, um eine durch Gasblasen in der Probenlösung über den Ausgleichskanal in die Druckkammer gedrückte Probenflüssigkeitsmenge zu verdampfen und dadurch einen gasblasenreduzierenden Gegendruck auf die in der Reaktionskammer vorliegende Probenlösung zu erzeugen.

Der Normalsiedepunkt bezeichnet dabei wie üblich den Siedepunkt der Probenlösung bei Normaldruck (1013,25 hP).

Die Druckkammer weist nur eine einzige Öffnung auf und sie steht mit dieser Öffnung mit der Reaktionskammer in Fluidverbindung.

In einer vorteilhaften Erfindungsvariante ist die Reaktionskammer mittels der ersten Heizeinrichtung auf eine Solltemperatur zwischen 10 K und 2 K unterhalb des Normalsiedepunkts der Probenlösung, insbesondere auf eine Solltemperatur zwischen 92 °C und 98°C heizbar. Die Heizeinrichtung kann Teil der mikrofluidischen Vorrichtung oder auch durch eine externe Einrichtung gebildet sein.

In einer besonders bevorzugten Gestaltung ist die Reaktionskammer der mikrofluidischen Vorrichtung zur Durchführung einer PCR, insbesondere einer Real Time PCR eingerichtet.

Vorteilhaft ist die Druckkammer mittels der zweiten Heizeinrichtung auf eine Druckkammertemperatur zwischen 10 K und 30 K oberhalb des Normalsiedepunkts der Probenlösung, insbesondere auf eine Druckkammertemperatur zwischen 110°C und 130°C heizbar. Auch hier kann die Heizeinrichtung Teil der mikrofluidischen Vorrichtung oder durch eine externe Einrichtung gebildet sein.

Bei vorteilhaften Erfindungsvarianten steht die Reaktionskammer in Fluidverbindung mit einer Einrichtung zum Nachweis von Biomolekülen in der Probenlösung. Die Vorrichtung kann mit Vorteil für ein zellbiologisches, analytisches und/oder diagnostisches System, insbesondere für ein Lab-on-Chip System vorgesehen sein.

Besondere Vorteile bietet die mikrofluidische Vorrichtung, wenn sie ein abgeschlossenes System bildet. Die mikrofluidische Vorrichtung ist besonders bevorzugt in einer mikrofluidischen Kartusche, insbesondere einer aus Polycarbonat bestehenden mikrofluidischen Kartusche ausgebildet.

Die beschriebene Druckkammer kann einfach in ein mikrofluidisches System integriert werden und erfordert für die Unterdrückung der Gasblasenbildung keine komplexe Auslegungen der Reaktionskammer, Zugaben zur Probenlösung oder andere aufwendige Maßnahmen, die in der Regel entweder ohnehin nur eine Nukleation von Gasblasen an den Reaktorwänden verhindern können oder, wie im Fall von Zugaben zur Probenlösung, den Ablauf der PCR beeinträchtigen können.

Die Erfindung enthält auch ein Verfahren zur temperaturgesteuerten Verarbeitung einer Probenlösung in einer Reaktionskammer, die über einen Ausgleichskanal mit einer Druckkammer in Fluidverbindung steht, welche nur eine einzige Öffnung aufweist und über diese Öffnung mit der Reaktionskammer in Fluidverbindung steht, wobei bei dem Verfahren
- die Probenlösung der Reaktionskammer zugeführt und auf eine Solltemperatur nahe des Normalsiedepunkts der Probenlösung geheizt wird, und
- die Druckkammer auf eine Temperatur oberhalb des Normalsiedepunkts der Probenlösung geheizt wird,
- so dass die Druckkammer im Normalbetrieb flüssigkeitsfrei ist und eine durch Gasblasen in der Probenlösung über den Ausgleichskanal in die Druckkammer gedrückte Probenflüssigkeitsmenge in der Druckkammer verdampft und dadurch einen gasblasenreduzierenden Gegendruck auf die in der Reaktionskammer vorliegende Probenlösung erzeugt.

Bei einer vorteilhaften Verfahrensführung wird die Reaktionskammer auf eine Solltemperatur zwischen 10 K und 2 K unterhalb des Normalsiedepunkts der Probenlösung, konkret beispielsweise auf eine Solltemperatur zwischen 92 °C und 98°C geheizt. Die Druckkammer wird alternativ oder ' zusätzlich auf eine Druckkammertemperatur zwischen 10 K und 30 K oberhalb des Normalsiedepunkts der Probenlösung, konkret beispielsweise auf eine Druckkammertemperatur zwischen 110°C und 130°C geheizt.

Vorteile der Erfindung sowie weitere Ausführungsbeispiele werden nachfolgend anhand der Figuren erläutert, bei deren Darstellung auf eine maßstabs- und proportionsgetreue Wiedergabe verzichtet wurde, um die Anschaulichkeit zu erhöhen.

Es zeigen:
- Fig. 1: eine schematische Darstellung des Prinzips des Nachweises von Nukleinsäuren in einer Probe durch ein Nachweissystem,
- Fig. 2: schematisch eine mikrofluidische Kartusche für den Nachweis von Biomolekülen in einer Probenlösung,
- Fig. 3: schematisch einen Querschnitt der Kartusche von Fig. 2 im Bereich des Mikrochips entlang der Linie III-III,
- Fig. 4: einen Ausschnitt einer mikrofluidischen Kartusche im Bereich der Nachweiskammer und der PCR-Kammer in perspektivischer Aufsicht,
- Fig. 5: einen Ausschnitt einer erfindungsgemäßen mikrofluidischen Vorrichtung zur temperaturgesteuerten Verarbeitung von Biomolekülen in einer Probenlösung,
- Fig. 6: schematisch im Querschnitt eine Doppelspritze, die als Pumpeinrichtung in einer mikrofluidischen Kartusche eingesetzt werden kann,
- Fig. 7: in (a) bis (c) die Doppelspritze der Fig. 6 in verschiedenen Zuständen bei der Zuführung/ Rückführung einer Flüssigkeit, und
- Fig. 8: in (a) bis (e) eine Mehrflüssigkeiten-Doppelspritze in verschiedenen Zuständen bei der Zuführung/Rückführung von Flüssigkeiten.

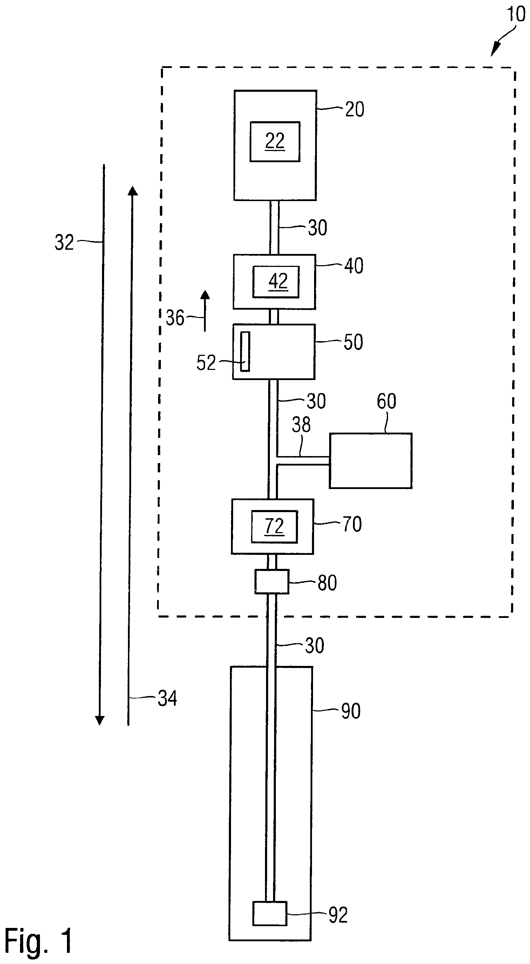

Figur 1 illustriert das Prinzip des Nachweises bestimmter Nukleinsäuren in einer in einem Probensystem 90 vorliegenden Probe 92 durch ein Nachweissystem 10. Bei der Probe 92 kann es sich beispielsweise um eine Abstrichprobe eines Patienten handeln, die am Ende eines Tupferstabes in einem Probenröhrchen 90 vorliegt und die mikrobiologisch auf MRSA-Stämme (Methicillin resistenter Staphylococcus aureus) untersucht werden soll.

Das Nachweissystem 10 enthält eine Pumpeinrichtung 20 mit einer Prozessflüssigkeit 22, die in einem Zuführungsschritt über ein Mikrokanalsystem 30 und ein offenes Ventil 80 der Probe 92 zugeführt wird (Flussrichtung 32). Auf ihrem Weg durch das Mikrokanalsystem 30 durchläuft die Prozessflüssigkeit 22 einen Vorratsbereich 70, in dem sie dort vorgelegte Chemikalien 72, im Ausführungsbeispiel etwa für die Freilegung der DNA der Probe 92, aufnimmt. Die Nachweiskammer 40, die Reaktionskammer 50 und die Druckkammer 60 des Nachweissystems 10 spielen bei diesem Zuführungsschritt noch keine Rolle.

Dann wird in einem Rückführungsschritt die durch die Lyse der Probe 92 in dem Probensystem 90 entstandene Probenlösung durch die Pumpeinrichtung 20 in das Nachweissystem 10 zurückgeführt (Flussrichtung 34). Dabei wird in der Regel die Probenlösung bis in die Pumpeinrichtung 20 zurückgezogen, um sicherzustellen, dass zumindest die Reaktionskammer 50 des Nachweissystems 10 mit Probenlösung gefüllt ist. Nach dem Rückführungsschritt wird das Ventil 80 zum externen Probensystem 90 geschlossen um ein definiertes Fluidvolumen im Nachweissystem 10 vorliegen zu haben und ein abgeschlossenes System zu erhalten.

Zur Amplifikation der nachzuweisenden Nukleinsäuren wird in der Reaktionskammer 50 eine Polymerase-Kettenreaktion (PCR) durchgeführt. Die dafür benötigten Chemikalien 52 liegen zunächst unter einer Wachsschicht geschützt vorkonfektioniert vor. Zum Start der Amplifikation wird die Wachsschicht durch eine Temperaturerhöhung geschmolzen und die PCR-Chemikalien 52 dadurch der Probenlösung in der Reaktionskammer 50 beigegeben. Während der PCR bleibt das geschmolzene Wachs flüssig bzw. wird von mehreren Reservoiren im Randbereich der Reaktionskammer 50 aufgenommen um sicherzustellen, dass kein verfestigtes Wachs angrenzende Mikrokanäle 30 verstopft.

Der für die PCR erforderliche zyklische Temperaturverlauf in der Reaktionskammer 50 wird mit Hilfe einer der Reaktionskammer 50 zugeordneten Heiz-/Kühleinrichtung (nicht gezeigt) erzeugt. Der in mikrofluidischen Systemen nicht selbstverständliche kontrollierte Ablauf der PCR wird in dem Nachweissystem 10 durch eine über einen Ausgleichskanal 38 mit der Reaktionskammer 50 in Fluidverbindung stehende Druckkammer 60 sichergestellt. Wie weiter unten genauer erläutert, ist die Druckkammer 60 im Normalbetrieb flüssigkeitsfrei und wird durch eine der Druckkammer zugeordnete Heizeinrichtung (nicht gezeigt) auf einer Temperatur deutlich oberhalb des Normalsiedepunkts der Probenlösung, beispielsweise T_{DK} = 120 °C, gehalten.

Eventuell in der Reaktionskammer 50 im Denaturierungsschritt bei T ≈ 96 °C entstehende Gasblasen drücken eine kleine Menge der Probenflüssigkeit in die Druckkammer 60, wo sie wegen der erhöhten Druckkammertemperatur sofort verdampft. Durch das bei der Verdampfung erzeugte große Gasvolumen entsteht in der Druckkammer 60 rasch ein Gegendruck auf die in der Reaktionskammer 50 vorliegende Probenlösung, der die Anzahl und Größe der Gasblasen stark vermindert. Die Reaktionskammer 50 und die Drucckammer 60 bilden so erfindungsgemäß ein selbstregulierendes Subsystem zur Unterdrückung unerwünschter Gasblasen in der Reaktionskammer 50 und gewährleisten dadurch einen wohl-kontrollierten Ablauf der PCR.

Konkret wird in dem Nachweissystem 10 eine besondere Umsetzungsart der PCR durchgeführt, die als Real Time PCR bezeichnet wird, da dabei die Menge an in der Probenlösung vorliegenden Nukleinsäure bestimmt wird, während die Reaktion abläuft. Die Real Time PCR erlaubt eine genauere Bestimmung der Konzentration der Nukleinsäure in der Probenlösung als eine reine Endpunktsbestimmung am Ende einer PCR. Bei der im Nachweissystem 10 durchgeführten Real Time PCR wird zunächst eine erste vorbestimmte Anzahl von Zyklen der Amplifikationsreaktion durchgeführt, beispielsweise 15 Zyklen, um die Konzentration an Nukleinsäure in den Messbereich anzuheben.

Anschließend wird mit Hilfe der Pumpeinrichtung 20 ein definiertes Volumen an Probenlösung mit amplifizierten Nukleinsäuren aus der Reaktionskammer 50 in die Nachweiskammer 40 überführt (Fluss 36). In der Nachweiskammer ist der Sensorbereich 42 eines CMOS-basierten Mikrochips angeordnet, der ein Array von funktionalisierten Teststellen zur elektrochemischen Detektion der Nukleinsäuren in der Probenlösung aufweist. Bei der elektrochemischen Detektion erzeugen die Teststellen jeweils ein elektrisches Signal, dessen Größe ein Maß für die Konzentration an Nukleinsäure in dem überführten Nachweisvolumen darstellt. Ein bevorzugtes elektrochemisches Detektionsverfahren ist in der Druckschrift DE 10 2011 056 606 B3 beschrieben.

Anschließend wird wiederholt jeweils ein weiterer PCR-Zyklus durchgeführt, um die Konzentration an Nukleinsäure weiter zu erhöhen und nach jedem Zyklus wird ein definiertes Volumen an Probenlösung mit amplifizierten Nukleinsäuren aus der Reaktionskammer 50 in die Nachweiskammer 40 überführt und die Konzentration an Nukleinsäure elektrochemisch bestimmt. Auf diese Weise können beispielsweise insgesamt 30 bis 40 PCR-Zyklen durchgeführt werden. Am Ende des Verfahrens wird zur Endpunktsbestimmung noch ein größeres Abschlussvolumen an Probenlösung in die Nachweiskammer 40 gezogen und die Konzentration an Nukleinsäure elektrochemisch detektiert.

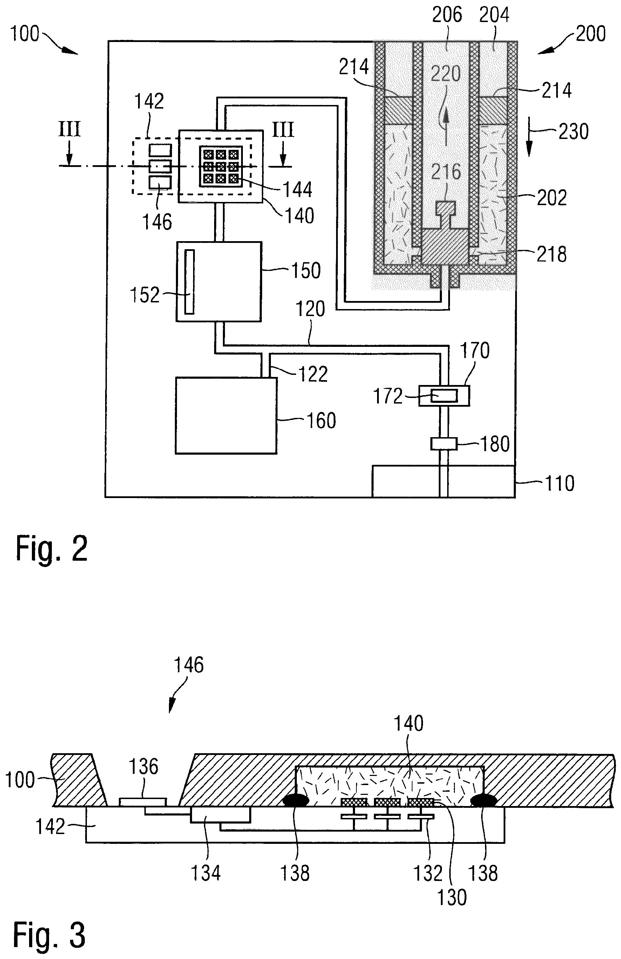

Als konkrete Umsetzung eines Nachweissystems 10 zeigt Fig. 2 schematisch ein Nachweissystem in Form einer mikrofluidischen Kartusche 100 für den Nachweis von Biomolekülen in einer Probenlösung, die entsprechend den im Zusammenhang mit Fig. 1 erläuterten Prinzipien aufgebaut ist.

Die Kartusche 100 ist bis auf die vorgelegten Chemikalien und den Mikrochip 142 vollständig aus Polycarbonat gebildet, beispielsweise durch ein additives Fertigungsverfahren oder durch Spritzguss. Die Kartusche 100 ist eine Einwegkartusche für einen kostengünstigen Nachweis gewünschter Nukleinsäuren, beispielsweise in einem MRSA-Screening.

Für die Verbindung mit einem Probensystem weist die Kartusche 100 einen Luer-Anschluss 110 auf, an den beispielsweise ein Probenröhrchen 90 mit einer zu untersuchenden Abstrichprobe 92 angeschlossen werden kann.

In der Kartusche 100 ist ein Mikrokanalsystem 120 ausgebildet, das sich von einer Pumpeinrichtung 200 über eine Nachweiskammer 140, eine PCR-Kammer 150 mit durch eine Wachsschicht geschützten PCR-Chemikalien 152 und einem Vorratsbereich 170 für Lysechemikalien 172 zu dem Luer-Anschluss 110 hin erstreckt. In anderen Gestaltungen sind die Lysechemikalien 172 an andere Stelle, beispielsweise im Luer-Anschluss oder im Probenröhrchen 90 vorgesehen.

Die Pumpeinrichtung ist in Form der weiter genauer unten erläuterten Doppelspritze 200 ausgestaltet und bildet einen integralen Bestandteil der Polycarbonat-Kartusche 100. Eine Außenkammer 204 der Doppelspritze 200 ist dabei lagerstabil mit einer gewünschten Prozessflüssigkeit 202 vorgefüllt, so dass die Kartusche 100 bis auf die Heiz-/Kühleinrichtung im Wesentlichen alle für den gewünschten Nachweis benötigten Komponenten enthält.

Zur Bestimmung der Konzentration der Nukleinsäuren in der Probenlösung enthält die Kartusche einen CMOS-basierten Mikrochip 142, dessen Umriss in Fig. 2 gestrichelt eingezeichnet ist. Der Mikrochip 142 weist einen in der Nachweiskammer 140 angeordneten Sensorbereich 144 und einen flüssigkeitsdicht von der Nachweiskammer abgetrennten Kontaktbereich 146 auf. Zur genaueren Erläuterung zeigt Fig. 3 schematisch einen Querschnitt der Kartusche 100 im Bereich des Mikrochips 142 entlang der Linie III-III von Fig. 2.

Der Sensorbereich 144 des Mikrochips 142 enthält ein Array von beispielsweise 109 funktionalisierten Teststellen 130 in oktagonaler Anordnung zur elektrochemischen Detektion der nachzuweisen Nukleinsäuren in der Probenlösung. Ein geeignetes Nachweisverfahren ist dabei beispielsweise in der Druckschrift DE 10 2011 056 606 B3 beschrieben.

Für ein besonders gutes Ausleseergebnis ist jede der Teststellen 130 des Sensorbereichs 144 mit einem eigenen Sigma-Delta-Modulator 132 erster Ordnung für die AD-Wandlung der an den Teststellen 130 erzeugten elektrischen Signale ausgestattet. Die Sigma-Delta-Modulatoren 132 liefern als digitalisiertes Signal einen Bitstrom, der unempfindlich gegenüber Rauschen und Übersprechen ist, der jedoch für die vollständige AD -Wandlung entsprechend der an sich bekannten Prinzipien der Sigma-Delta-Technik in einer Verarbeitungsschaltung 134 zur Erzeugung der endgültigen Ausgangssignale noch weiterverarbeitet wird. Die Ausgangssignale können an den Kontaktpads 136 des Kontaktbereichs 146 des Mikrochips 142 abgegriffen und in bekannter Weise einer Auswerte- und Anzeigeeinheit zugeführt werden.

Wie in Fig. 3 gezeigt, ist der Kontaktbereich 146 des Mikrochips 142 beispielsweise mittels eines Gummirings 138 oder einer Vergussmasse flüssigkeitsdicht von der Nachweiskammer 140 mit der darin im Einsatz enthaltenen Probenlösung abgetrennt.

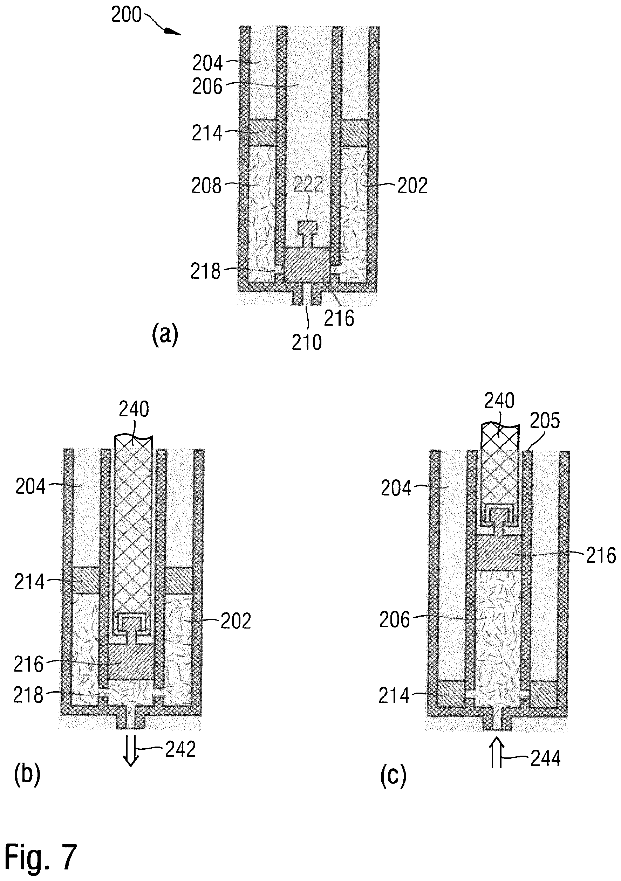

Zur Untersuchung einer Probe 92 wird die Kartusche 100 zunächst über den Luer-Anschluss 110 mit dem Probensystem 90 verbunden und das Ventil 180, sofern nicht bereits offen, geöffnet. Nun können durch ein leichtes Anheben 220 des Innenkolbens 216 der Doppelspritze 200 mit einer zugehörigen Kolbenstange (Fig. 7(b)) die Durchgangsbohrungen 218 zwischen Außenkammer und Innenkammer 206 freigegeben werden. Durch ein Herabdrücken 230 des Gummirings 214 wird die vorgelegte Prozessflüssigkeit 202 über das Mikrokanalsystem 120 der Probe 92 zugeführt, wobei die Prozessflüssigkeit im Vorratsbereich 170 die Lysechemikalien 172 aufnimmt.

Der herabgedrückte Gummiring 214 verschließt die Durchgangsbohrungen 218 von der Außenkammerseite her, so dass durch eine weitere axiale Bewegung des Innenkolbens 216 mit der Kolbenstange die durch die Lyse der Probe 92 entstandene Probenlösung in das Mikrokanalsystem 120 der Kartusche 100 bis in die Innenkammer 206 der Doppelspritze 200 zurückgezogen werden kann. In diesem Zustand ist das Kanalsystem 120 einschließlich der PCR-Kammer 150 und der Nachweiskammer 140 mit Probenlösung gefüllt. Die über den Ausgleichskanal 122 in Fluidverbindung mit der PCR-Kammer 150 stehende Druckkammer 160 bleibt dagegen im Wesentlichen flüssigkeitsfrei. Dann wird das Ventil 180 zum externen Probensystem 90 geschlossen um für die nachfolgende PCR ein definiertes eingeschlossenes Fluidvolumen im Nachweissystem zu haben.

Für die Durchführung der PCR kann die Kartusche 100 beispielsweise in einen Thermocycler eingesetzt werden, der Heiz-/ Kühleinrichtungen für die Temperaturschritte der PCR-Zyklen bereitstellt. Insbesondere werden in der PCR-Kammer 150 durch Schmelzen der Wachsschicht die PCR-Chemikalien 152 der Probenlösung beigegeben und die PCR durch Erzeugen des gewünschten zyklischen Temperaturverlaufs durchgeführt. Wie weiter unten genauer beschrieben wird zudem durch eine Aufheizung der Druckkammer 160 auf eine Temperatur T_{DK} oberhalb der Siedetemperatur der Probenlösung eine Gasblasenbildung in der PCR-Kammer 150 unterdrückt, so dass die PCR zuverlässig und kontrolliert ablaufen kann.

Nach einer vorbestimmten Anzahl an initialen PCR-Zyklen wird durch kontrolliertes Anheben des Innenkolbens 216 der Doppelspritze ein definiertes Volumen an Probenlösung mit amplifizierten Nukleinsäuren aus der PCR-Kammer 150 in die Nachweiskammer 140 überführt. Die gewünschten Konzentrationswerte an Nukleinsäure in der Probenlösung werden durch elektrochemische Detektion der Nukleinsäureoligomer-Hybridisierungsereignissen an den Teststellen 130 des Mikrochips 142 bestimmt, wie beispielsweise in der DE 10 2011 056 606 B3 genauer beschrieben.

Nach jeweils einem weiteren PCR-Zyklus wird durch sukzessives Anheben des Innenkolbens 216 jeweils ein weiteres definiertes Volumen an weiter amplifizierter Probenlösung aus der PCR-Kammer 150 in die Nachweiskammer 140 überführt und die Konzentration an Nukleinsäure bestimmt. Beispielsweise kann die PCR-Kammer 150 ein Volumen von 30 µl aufweisen und es wird nach Abschluss der initialen PCR-Zyklen für 15 bis 25 Zyklen nach jedem PCR-Zyklus ein Volumen von 1 µl in die Nachweiskammer 140 überführt. Am Ende des Verfahrens kann noch ein größeres Abschlussvolumen an Probenlösung in die Nachweiskammer 140 gezogen und ein Konzentationsendpunkt bestimmt werden. Es versteht sich, dass die Konzentrationsbestimmung auch in größeren Abständen, beispielsweise nach jedem zweiten oder jedem dritten PCR-Zyklus durchgeführt werden kann.

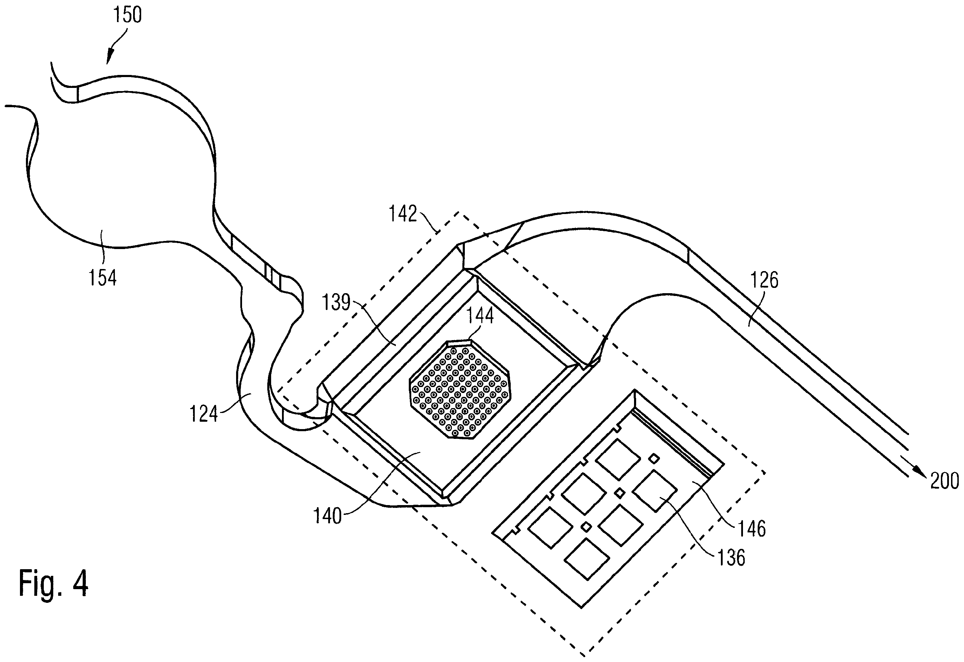

Eine konkrete Gestaltung der Kartusche 100 im Bereich der Nachweiskammer 140 und der PCR-Kammer 150 ist in Fig. 4 in perspektivischer Aufsicht gezeigt. Bei dieser Gestaltung ist die PCR-Kammer 150 in mehrere nacheinander angeordnete, scheibenförmige Teilkammern 154 unterteilt, von denen in der Figur nur die letzte Teilkammer 154 dargestellt ist. Von der PCR-Kammer 150 bzw. der letzten Teilkammer 154 führt ein Zuleitungskanal 124 in die Nachweiskammer 140, von der sich ein Auslasskanal 126 zur Doppelspritze 200 hin erstreckt.

Der Umriss des CMOS-basierten Mikrochips 142 ist wieder gestrichelt eingezeichnet. Dabei ist der Sensorbereich 144 des Mikrochips 142 mit dem Array von Teststellen 130 in der Nachweiskammer 140 angeordnet und über einen umlaufenden Vergussmassenring 139 flüssigkeitsdicht von dem Kontaktbereich 146 mit den Kontaktpads 136 abgetrennt.

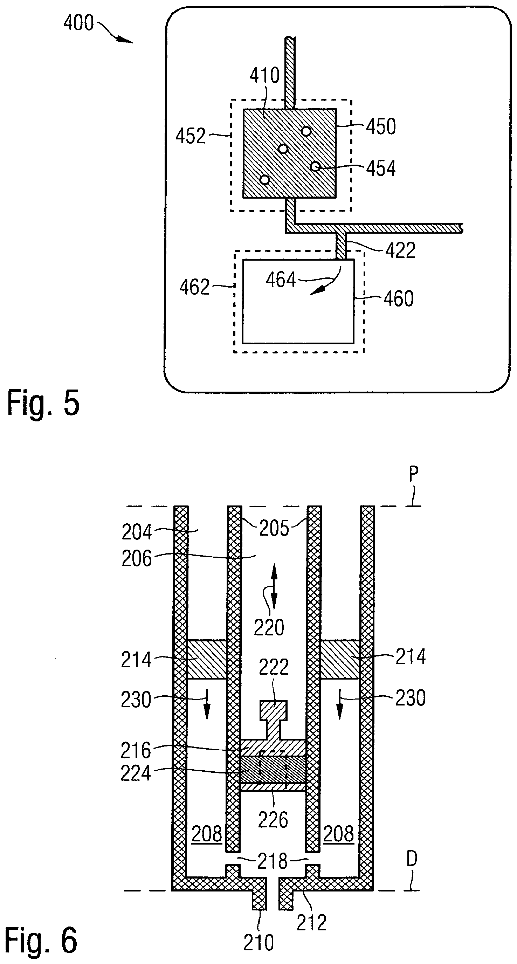

Das selbstregulierende Subsystem zur Unterdrückung von Gasblasen in der PCR-Kammer ist in Fig. 5 nochmals genauer erläutert. Das beschriebene Prinzip ist nicht auf die Anwendung in einer PCR beschränkt, sondern kann auch bei anderen temperaturgesteuerten mikrofluidischen Verarbeitungsprozessen eingesetzt werden, bei denen eine Probenlösung nahe des Normalsiedepunkts der Probenlösung gasblasenfrei gehalten werden soll.

Figur 5 zeigt einen Ausschnitt einer erfindungsgemäßen mikrofluidischen Vorrichtung 400 zur temperaturgesteuerten Verarbeitung einer Probenlösung 410, hier dem oben beschriebenen Nachweis von Nukleinsäuren. Die mikrofluidische Vorrichtung 400 enthält eine Reaktionskammer 450, in der die Probenlösung mit den Nukleinsäuren durch eine Heiz-/Kühleinrichtung 452 auf eine Solltemperatur nahe des Normalsiedepunkts der Probenlösung 410 heizbar ist. Bei der oben angesprochenen Real Time PCR wird die Probenlösung 410 beispielsweise im Denaturierungsschritt auf etwa 96 °C geheizt.

Erfindungsgemäß werden Gasblasen in der Reaktionskammer 450 durch eine fluidische Ankoppelung der Reaktionskammer 450 an eine im Normalbetrieb flüssigkeitsfreie Druckkammer 460 mit einer Temperatur oberhalb des Normalsiedepunkts der Probenlösung 410 unterdrückt. Wie in Fig. 5 dargestellt, steht die Reaktionskammer 450 über einen Ausgleichskanal 422 mit einer Druckkammer 460 in Fluidverbindung, welche mittels einer Heizeinrichtung 462 auf eine Temperatur T_{DK} deutlich oberhalb des Normalsiedepunkts der Probenlösung geheizt wird. Beispielsweise liegt die Drucckammertemperatur zwischen 110 °C und 130 °C, konkret etwa bei T_{DK} = 125 °C. Da die Druckkammertemperatur oberhalb der Siedetemperatur liegt, ist die Druckkammer 460 im Normalbetrieb flüssigkeitsfrei.

Ohne an eine bestimmte Erklärung gebunden sein zu wollen, wird der Mechanismus der Gasblasen-Unterdrückung gegenwärtig wie folgt verstanden: Falls bei der temperaturgesteuerten Verarbeitung der Biomoleküle, wie etwa dem oben genannten PCR-Denaturierungsschritt Gasblasen 454 in der Reaktionskammer 450 entstehen, so wird durch die ersten sich bildenden Gasblasen eine kleine Probenflüssigkeitsmenge 464 über den Ausgleichskanal 422 in die Druckkammer 460 gedrückt, wo sie aufgrund der in der Druckkammer 460 herrschenden hohen Temperatur sofort verdampft. Das durch die Verdampfung entstehende Gasvolumen ist 1000 bis 2000 mal größer als das Volumen der verdampften Flüssigkeit, so dass ein starker Druckanstieg in der Druckkammer 460 resultiert, der seinerseits zu einer Druckerhöhung in der Reaktionskammer 450 und damit zu einer Reduzierung der Anzahl und Größe der Gasblasen führt und der Bildung weiterer Gasblasen entgegenwirkt. Die Haupteffekte sind nach gegenwärtigem Verständnis dabei die Erhöhung des Dampfdrucks und die Komprimierung der vorhandenen Gasvolumina.

Da die Stärke des erzeugten Gegendrucks mit der verdampften Flüssigkeitsmenge und damit mit dem von den Gasblasen verdrängten Flüssigkeitsvolumen 464 zunimmt, führt diese Vorgehensweise zu einer Selbstregulierung der Gasblasen 454 in der Probenlösung 410. Die Druckkammer 460 kann einfach in ein mikrofluidisches System integriert werden und bietet die oben beschriebenen Vorteile gegenüber herkömmlichen Lösungen.

Für die Zuführung von Prozessflüssigkeit 202 in ein Mikrofluidiksystem, wie etwa das Mikrokanalsystem 120,140, 150, 170, 180 der Fig. 2 wird mit Vorteil eine Doppelspritze 200 eingesetzt, deren Bauweise und Funktionsprinzip nunmehr mit Bezug auf Fig. 6 näher erläutert wird.

Die Doppelspritze 200 enthält zwei konzentrisch angeordneten Zylinderkammern 204, 206, in denen jeweils axial bewegliche Kolben 214, 216 aufgenommen sind. In dieser Beschreibung wird das die Auslassöffnung 210 aufweisende Ende der Doppelspritze 200 als distales Ende D des Spritze bzw. der Zylinder bezeichnet, das axial entgegengesetzte Ende als proximales Ende P. Genauer enthält die Doppelspritze eine zylindrische Außenkammer 204, die einen Vorratsraum 208 für die Prozessflüssigkeit 202 definiert. Konzentrisch in der Außenkammer 204 ist eine zylindrische Innenkammer 206 angeordnet, die an ihrem distalen Ende mit der Außenkammer 204 abschließt. Die Auslassöffnung 210 der Doppelspritze ist in der distalen Zylinder-Deckfläche 212 der Innenkammer 206 angeordnet. Die beiden Zylinderkammern 204, 206 stehen über eine Reihe von Durchgangsbohrungen 218 miteinander in Fluidverbindung, welche im distalen Endbereich der Kammern 204, 206 auf gleicher axialer Höhe umlaufend in der gemeinsamen Zylinderwand 205 von Innen- und Außenkammer ausgebildet sind.

In der Innenkammer 206 ist ein axial beweglicher Innenkolben 216 aufgenommen, der an seiner proximalen Seite ein Griffelement 222 aufweist, das von außen mit einer separaten, angepassten Kolbenstange 240 (Fig. 7(b)) gegriffen werden kann, um den Innenkolben 216 axial auf- und ab bewegen zu können (Pfeile 220). Anstelle des Griffelements 222 kann beispielsweise auch ein Hohlraum in der proximalen Seite des Innenkolbens 216 ausgebildet sein, in den das entsprechend ausgestaltete distale Ende einer Kolbenstange eingreift.

Befindet sich der Innenkolben 216 am distalen Ende der Innenkammer 206, so verschließt der Innenkolben 216 mit einer umlaufenden Dichtung 224, beispielsweise einer Gummidichtung, die Durchgangsbohrungen 218 von der Seite der Innenkammer her, so dass kein Fluidfluss von dem Vorratsraum 208 der Außenkammer 204 zur Auslassöffnung 210 hin möglich ist. Diese Stellung des Innenkolbens 216 wird daher als Verschlussstellung bezeichnet und ist in Fig. 7(a) gezeigt.

Wird der Innenkolben 216 etwas nach proximal bewegt, in der Orientierung der Fig. 6 also angehoben, so gibt er die Durchgangsbohrungen 218 frei und ermöglicht einen Fluidfluss von der Außenkammer 204 über die Innenkammer 206 zur Auslassöffnung 210. Eine solche Stellung des Innenkolbens 216 wird daher als Freigabestellung bezeichnet und ist in Fig. 7(b) gezeigt.

In der Außenkammer 204 ist ein die Innenkammer 206 umlaufender ringförmiger dichtender Außenkolben, hier in Form eines Gummirings 214 aufgenommen, der axial zu dem distalen Ende D der Außenkammer hin beweglich ist (Pfeile 230). In einer proximalen Vorratsstellung schließt der Gummiring 214 den Vorratsraum 208 für die Prozessflüssigkeit 202 ab, wie in Fig. 7(a) dargestellt. In einer distalen Verschlussstellung, die in Fig. 7(c) gezeigt ist, verschließt der Gummiring 214 die Durchgangsbohrungen 218 von der Seite der Außenkammer 204 her, und ermöglicht so durch eine axiale Bewegung des Innenkolbens 216 zum proximalen Ende P der Spritze hin ein Rückziehen von Flüssigkeit aus dem Mikrofluidsystem in die Innenkammer 206.

Auf der proximalen Seite des Innenkolbens 216 kann ein Vorratsraum 226 für die Aufnahme von Chemikalien vorgesehen sein. Die Chemikalien sind dabei zunächst in dem Vorratsraum 226 fixiert, können aber durch eine Releasefunktion, beispielsweise eine schmelzbare Wachsabdeckung oder eine magnetische Partikel-Deckschicht zu einem gewünschten Zeitpunkt freigegeben werden.

Die Funktionsweise der Doppelspritze 100 wird nun anhand der Figuren 7(a) bis 7(c) näher erläutert, bei denen eine in der Außenkammer 204 der Doppelspritze 200 vorgelegte Prozessflüssigkeit 202 einem selbst nicht dargestellten Mikrofluidiksystem zugeführt wird.

Im Ausgangszustand der Fig. 7(a) befindet sich der Innenkolben 216 in seiner distalen Verschlussstellung und der Gummiring 214 in seiner proximalen Vorratsstellung. Der Vorratsraum 208 der Außenkammer 206 ist mit der gewünschten Prozessflüssigkeit 202 lagerstabil vorgefüllt.

Soll die Prozessflüssigkeit 202 dem Mikrofluidiksystem zugeführt werden, so wird das Griffelement 222 des Innenkolbens 216 mit einer angepassten Kolbenstange 240 gegriffen und in die in Fig. 7(b) gezeigte Freigabestellung angehoben, in der die Durchgangsbohrungen 218 den Fluidfluss von dem Vorratsraum 208 der Außenkammer 204 zur Auslassöffnung 210 freigeben. Durch ein Herabdrücken des Gummirings 214 kann die Prozessflüssigkeit 202 vollständig in das Mikrofluidiksystem gepumpt werden (Fluss 242).

Wie in Fig. 7(c) dargestellt, sind der Gummiring 214 und die Position der Durchgangsöffnungen 218 in der Zylinderwand 205 so aufeinander abgestimmt, dass der Gummiring 214 in seiner distalen Verschlussstellung die Durchgangsbohrungen 218 von der Außenkammerseite her verschließt. In diesem Zustand kann die Doppelspritze durch ein axiales Auf- und Abbewegen der Kolbenstange 240 in der Innenkammer 206 wie eine gewöhnliche Spritze benutzt und Flüssigkeit in beide Richtungen bewegt werden. Insbesondere kann durch ein weiteres Anheben, also eine axiale Bewegung des Innenkolbens 216 zum proximalen Ende hin Flüssigkeit aus dem Mikrofluidsystem in die Innenkammer 206 gezogen werden (Fluss 244).

Auf diese Weise wird beispielsweise bei dem Nachweissystem der Fig. 2 die durch Lyse der Probe 92 entstandene Probenlösung aus dem Probenröhrchen 90 in das Mikrokanalsystem der Kartusche 100 und letztlich in die Innenkammer 206 der Doppelspritze zurückgezogen. Auch während der Durchführung der PCR werden nach jedem PCR-Zyklus durch kontrolliertes Anheben der Kolbenstange 240 sukzessive kleine Flüssigkeitsvolumina von der PCR-Kammer 150 in die Nachweiskammer 140 überführt.

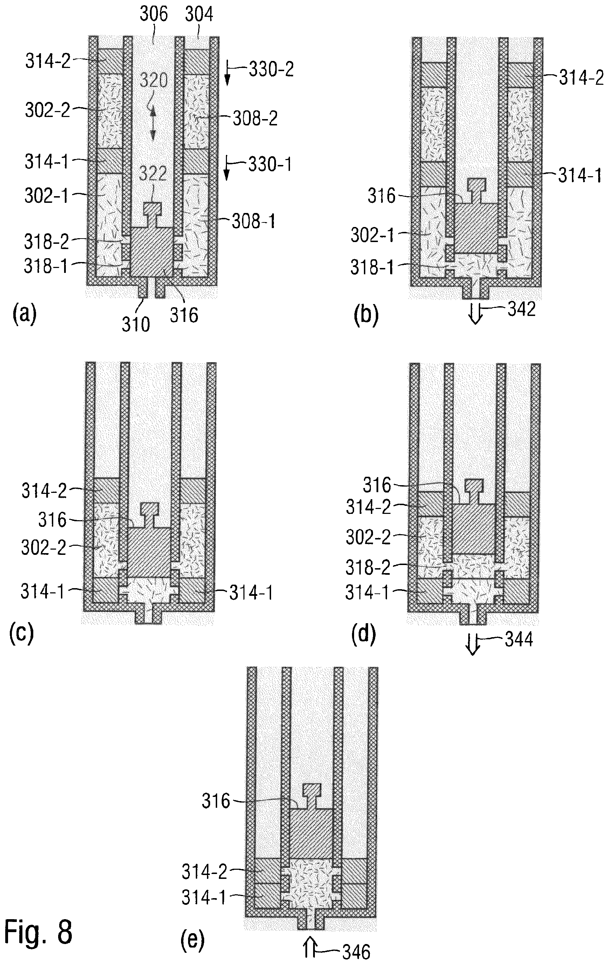

Mit einer solchen Doppelspritze können bei geeigneter Auslegung auch zwei oder mehr Flüssigkeiten einem Mikrofluidiksystem zugeführt werden. Figur 8 zeigt eine solche Mehrflüssigkeiten-Doppelspritze, die beispielhaft für die Zuführung von *n* = 2 Flüssigkeiten in ein Mikrofluidiksystem ausgelegt ist. Die Erweiterung auf die Zuführung von drei und mehr Flüssigkeiten ergibt sich aus der nachfolgenden Darstellung zwanglos.

Auch die 2-Flüssigkeiten-Doppelspritze 300 der Fig. 8 enthält zwei konzentrisch angeordneten Zylinderkammern 304, 306 und stellt daher eine Doppelspritze dar. Die zylindrische Außenkammer 304 definiert *n* = 2 Vorratsräume 308-1, 308-2 für die Aufnahme der zuzuführenden Flüssigkeiten 302-1, 302-2.

Konzentrisch in der Außenkammer 304 ist eine zylindrische Innenkammer 306 angeordnet, die an ihrem distalen Ende mit der Außenkammer 304 abschließt. Die Auslassöffnung 310 der Doppelspritze 300 ist in der distalen Zylinder-Deckfläche der Innenkammer 306 angeordnet.

Die beiden Zylinderkammern 304, 306 stehen über *n* = 2, in axialer Richtung beabstandete Gruppen von Durchgangsbohrungen 318-1, 318-2 miteinander in Fluidverbindung. Jede Gruppe von Durchgangsbohrungen 318-1, 318-2 besteht dabei aus einer Reihe von auf gleicher axialer Höhe umlaufend in der gemeinsamen Zylinderwand 305 von Innen- und Außenkammer ausgebildeten Durchgangsbohrungen.

In der Innenkammer 306 ist ein axial beweglicher Innenkolben 316 aufgenommen, der an seiner proximalen Seite ein Griffelement 322 aufweist, das von außen mit einer separaten, angepassten Kolbenstange gegriffen werden kann, um den Innenkolben 316 axial auf- und ab bewegen zu können (Pfeile 320). Auch hier kann anstelle des Griffelements 322 beispielsweise ein Hohlraum in der proximalen Seite des Innenkolbens 316 ausgebildet sein, in den das entsprechend ausgestaltete distale Ende einer Kolbenstange eingreift. Befindet sich der Innenkolben 316, wie in der in Fig. 8(a) gezeigten Ausgangsstellung am distalen Ende der Innenkammer 306, so verschließt der Innenkolben 316 mit einer umlaufenden Gummidichtung die *n* =2 Gruppen von Durchgangsbohrungen 318-1, 318-2 von der Seite der Innenkammer her, so dass kein Fluidfluss von den Vorratsräumen der Außenkammer 304 zur Auslassöffnung 310 hin möglich ist. Diese Stellung des Innenkolbens 316 wird daher als Verschlussstellung bezeichnet.

Wird der Innenkolben 316 etwas nach proximal bewegt, so gibt er zunächst nur die erste Gruppe von Durchgangsbohrungen 318-1 frei und ermöglicht einen Fluidfluss der ersten Flüssigkeit 302-1 von der Außenkammer 304 über die Innenkammer 306 zur Auslassöffnung 310. Diese Stellung des Innenkolbens 316 wird als erste Freigabestellung bezeichnet und ist in Fig. 8(b) illustriert. Durch eine weitere proximale Bewegung erreicht der Innenkolben 316 die zweite Freigabestellung, in der auch die zweite Gruppe von Durchgangsbohrungen 318-2 von der Innenkammerseite her freigegeben ist (siehe Fig. 8(d)).

Im allgemeinen Fall gibt es *n* axial beabstandete Freigabestellungen in denen der Innenkolben 316 sukzessive eine zunehmende Anzahl *k* = 1, ... , *n* von Gruppen von Durchgangsbohrungen freigibt und einen entsprechenden Fluidfluss von der Außenkammer in die Innenkammer ermöglicht.

In der Außenkammer 304 sind *n* =2 die Innenkammer 306 umlaufende, axial beabstandete Gummiringe 314-1, 314-2 aufgenommen, die jeweils axial zu dem distalen Ende D der Außenkammer hin beweglich sind (Pfeile 330-1, 330-2). In einer proximalen Vorratsstellung schließen die Gummiringe 314-1, 314-2 jeweils einen Vorratsraum für eine der zuführbaren Flüssigkeit 302-1, 302-2 ab, wie in Fig. 8(a) gezeigt.

In einer distalen Verschlussstellung verschließt der erste Gummiring 314-1 die erste Gruppe von Durchgangsbohrungen 318-1 (siehe Fig. 8(c)), während der zweite Gummiring 314-2 in seiner distalen Verschlussstellung die zweite Gruppe von Durchgangsbohrungen 318-2 verschließt (siehe Fig. 8(e)). Befindet sich der Innenkolben 316 in seiner ersten bzw. zweiten Freigabestellung, so kann durch die Bewegung der beiden Gummiringe 314-1, 314-2 bzw. nur des zweiten Gummirings 314-2 zum distalen Ende hin sukzessive die im ersten bzw. zweiten Vorratsraum vorgelegte Flüssigkeit über die erste bzw. zweite Gruppe von Durchgangsbohrungen 318-1, 318-2 in die Innenkammer 306 gedrückt werden. Sind beide Gummiringe 314-1, 314-2 in ihre distale Verschlussstellung gebracht, so sind alle Durchgangsbohrungen verschlossen und die Flüssigkeit kann durch eine proximale Bewegung des Innenkolbens 316 aus dem Mikrofluidiksystem in die Innenkammer 306 zurückgezogen werden.

Ausgehend von Ausgangszustand der Fig. 8(a) wird das Griffelement 322 des Innenkolbens 316 mit einer angepassten Kolbenstange gegriffen und in die erste Freigabestellung (Fig. 8(b)) angehoben. Durch ein Herabdrücken des beiden Gummiringe 314-1, 314-2 kann die erste Prozessflüssigkeit 302-1 vollständig in das Mikrofluidiksystem gepumpt werden (Flussrichtung 342). Dabei genügt es wegen der Inkompressibilität der Flüssigkeiten, den obersten der *n* Gummiringe, hier den Gummiring 314-2 aktiv nach distal zu drücken. Der ausgeübte Druck wird über die inkompressible zweite Prozessflüssigkeit 302-2 auf den ersten Gummiring 314-1 übertragen, so dass sich die beiden Gummiringe im gleichbleibenden Abstand nach distal bewegen und das Volumen des Vorratsraums 308-2 bei der Zuführung der ersten Prozessflüssigkeit 302-1 unverändert bleibt (vergleiche Fig. 8(a) und 8(c)).

Der erste Gummiring 314-1 und die Position der ersten Gruppe von Durchgangsöffnungen 318-1 in der Zylinderwand 305 sind so aufeinander abgestimmt, dass der erste Gummiring 314-1 in seiner distalen Verschlussstellung die erste Gruppe von Durchgangsbohrungen 318-1 von der Außenkammerseite her verschließt, wie in Fig. 8(c) gezeigt.

Nun kann der Innenkolben 316 weiter in die zweite Freigabestellung (Fig. 8(d)) angehoben werden, und durch ein Herabdrücken des zweiten Gummirings 314-2 die zweite Prozessflüssigkeit 302-2 vollständig in das Mikrofluidiksystem gepumpt werden (Flussrichtung 344). Dabei sind der zweite Gummiring 314-2 und die Position der zweiten Gruppe von Durchgangsöffnungen 318-2 in der Zylinderwand 305 so aufeinander abgestimmt, dass der zweite Gummiring 314-2 in seiner distalen Verschlussstellung die zweite Gruppe von Durchgangsbohrungen 318-1 von der Außenkammerseite her verschließt, wie in Fig. 8(e) gezeigt.

Da sich nunmehr alle Gummiringe 314-1, 314-2 in ihrer distalen Verschlussstellung befinden, kann die Doppelspritze 300 durch ein axiales Auf- und Abbewegen des Innenkolbens 316 mit der Kolbenstange wie eine gewöhnliche Spritze benutzt und Flüssigkeit in beide Richtungen bewegt werden. Insbesondere kann durch ein weiteres Anheben, also eine axiale Bewegung des Innenkolbens 316 zum proximalen Ende hin, Flüssigkeit aus dem Mikrofluidsystem in die Innenkammer 306 gezogen werden (Flussrichtung 246).

Bei einer Mehrflüssigkeiten-Doppelspritze mit n Ringkolben müssen die ersten *n*-*1* distalen Ringkolben nicht zwingend dichtend sein. Beispielsweise kann der Ringkolben 314-1 der Doppelspritze 300 zur Reibungsverminderung mit etwas Spiel zu den Zylinderwänden der Innen- bzw. Außenkammer ausgebildet sein, während der proximale Ringkolben 314-2 dichtend ausgebildet ist, um die Vorratsräume für die zuzuführenden Flüssigkeiten 302-1, 302-2 lagerstabil abzuschließen. In der Praxis findet eine Diffusion bzw. Mischung der Flüssigkeiten über einen schmalen Spielspalt des Ringkolbens 314-1 nicht statt oder ist jedenfalls vernachlässigbar gering.

In einer einfacheren Gestaltung muss es auch nicht *n* axial beabstandete Gruppen von Durchgangsbohrungen und *n* axial beabstandete Freigabestellungen geben. Vielmehr genügt bereits, wenn die Innenkammer über eine oder mehrere, in axialer Richtung beabstandete Gruppen von Durchgangsbohrungen mit der Außenkammer in Fluidverbindung steht und der Innenkolben in einer Verschlussstellung die eine oder mehreren Gruppen von Durchgangsbohrungen auf der Innenkammerseite verschließt und in einer oder mehreren Freigabestellungen einen Fluidfluss von der Außenkammer in die Innenkammer ermöglicht. Auch in einer solchen einfacheren Gestaltung kann durch die Bewegung der Ringkolben in der Außenkammer zum distalen Ende hin sukzessive eine im *k*-ten Vorratsraum vorgelegte Flüssigkeit über Durchgangsbohrungen in die Innenkammer gedrückt werden, wenn sich der Innenkolben in einer entsprechenden Freigabestellung befindet. Liegen weniger als *n* Gruppen von Durchgangsbohrungen vor, muss auch nicht jeder der *n* Ringkolben eine Gruppe von Durchgangsbohrungen verschließen, es muss lediglich sichergestellt sein, dass die *n* Ringkolben in ihrer distalen Verschlussstellung zusammen die eine oder mehreren Gruppen von Durchgangsbohrungen von der Außenkammerseite her verschließen, so dass ein Rückziehen von Flüssigkeit aus dem Mikrofluidsystem in die Innenkammer durch eine axiale Bewegung des Innenkolbens zum proximalen Ende hin ermöglicht ist, wenn alle *n* Ringkolben in ihre distale Verschlussstellung gebracht sind.

## Patentansprüche

1. Anordnung mit einer mikrofluidischen Vorrichtung (400) zur temperaturgesteuerten Verarbeitung einer Probenlösung und mit einer ersten und einer zweiten Heizeinrichtung (452, 462), wobei die mikrofluidische Vorrichtung (400) eine Reaktionskammer (450) aufweist, in der die Probenlösung mittels der ersten Heizeinrichtung (452) auf eine Solltemperatur nahe des Normalsiedepunkts heizbar ist, wobei die Reaktionskammer (450) über einen Ausgleichskanal (422) mit einer im Normalbetrieb flüssigkeitsfreien Druckkammer (460) in Fluidverbindung steht, welche mittels der zweiten Heizeinrichtung (462) auf eine Temperatur oberhalb des Normalsiedepunkts der Probenlösung heizbar ist, und die ausgelegt ist, um eine durch Gasblasen in der Probenlösung über den Ausgleichskanal (422) in die Druckkammer (460) gedrückte Probenflüssigkeitsmenge zu verdampfen und dadurch einen gasblasenreduzierenden Gegendruck auf die in der Reaktionskammer (450) vorliegende Probenlösung zu erzeugen, und wobei die Druckkammer (460) nur eine einzige Öffnung aufweist und sie über diese Öffnung mit der Reaktionskammer (450) in Fluidverbindung steht.

2. Anordnung nach Anspruch 1, **dadurch gekennzeichnet, dass** die Reaktionskammer (450) mittels der ersten Heizeinrichtung (452) auf eine Solltemperatur zwischen 10 K und 2 K unterhalb des Normalsiedepunkts der Probenlösung, insbesondere auf eine Solltemperatur zwischen 92 °C und 98°C heizbar ist.

3. Anordnung nach einem der Ansprüche 1 bis 2, **dadurch gekennzeichnet, dass** die Reaktionskammer (450) zur Durchführung einer PCR, insbesondere einer Real Time PCR eingerichtet ist.

4. Anordnung nach einem der Ansprüche 1 bis 3, **dadurch gekennzeichnet, dass** die Druckkammer (460) mittels der zweiten Heizeinrichtung (462) auf eine Druckkammertemperatur zwischen 10 K und 30 K oberhalb des Normalsiedepunkts der Probenlösung, insbesondere auf eine Drucckammertemperatur zwischen 110°C und 130°C heizbar ist.

5. Anordnung nach einem der Ansprüche 1 bis 4, **dadurch gekennzeichnet, dass** die Reaktionskammer (450) in Fluidverbindung mit einer Einrichtung zum Nachweis von Biomolekülen in der Probenlösung steht.

6. Anordnung nach einem der Ansprüche 1 bis 5, **dadurch gekennzeichnet, dass** die mikrofluidische Vorrichtung für ein zellbiologisches, analytisches und/oder diagnostisches System, insbesondere für ein Lab-on-Chip System vorgesehen ist.

7. Anordnung nach einem der Ansprüche 1 bis 6, **dadurch gekennzeichnet, dass** die mikrofluidische Vorrichtung ein abgeschlossenes System bildet.

8. Anordnung nach einem der Ansprüche 1 bis 7, **dadurch gekennzeichnet, dass** die mikrofluidische Vorrichtung in einer mikrofluidischen Kartusche, insbesondere einer aus Polycarbonat bestehenden mikrofluidischen Kartusche ausgebildet ist.

9. Anordnung nach einem der Ansprüche 1 bis 8, **dadurch gekennzeichnet, dass** die erste und zweite Heizeinrichtung (452, 462) gegenüber der mikrofluidischen Vorrichtung (400) externe Einrichtungen bilden.

10. Anordnung nach einem der Ansprüche 1 bis 8, **dadurch gekennzeichnet, dass** die erste und zweite Heizeinrichtung (452, 462) Teil der mikrofluidischen Vorrichtung (400) sind.

11. Verfahren zur temperaturgesteuerten Verarbeitung einer Probenlösung in einer Reaktionskammer, die über einen Ausgleichskanal mit einer Druckkammer in Fluidverbindung steht, welche nur eine einzige Öffnung aufweist und über diese Öffnung mit der Reaktionskammer in Fluidverbindung steht, wobei bei dem Verfahren
- die Probenlösung der Reaktionskammer zugeführt und auf eine Solltemperatur nahe des Normalsiedepunkts der Probenlösung geheizt wird, und
- die Druckkammer auf eine Temperatur oberhalb des Normalsiedepunkts der Probenlösung geheizt wird,
- so dass die Druckkammer im Normalbetrieb flüssigkeitsfrei ist und eine durch Gasblasen in der Probenlösung über den Ausgleichskanal in die Druckkammer gedrückte Probenflüssigkeitsmenge in der Druckkammer verdampft und dadurch einen gasblasenreduzierenden Gegendruck auf die in der Reaktionskammer vorliegende Probenlösung erzeugt.

12. Verfahren nach Anspruch 11, **dadurch gekennzeichnet, dass** die Reaktionskammer auf eine Solltemperatur zwischen 10 K und 2 K unterhalb des Normalsiedepunkts der Probenlösung, insbesondere auf eine Solltemperatur zwischen 92 °C und 98°C geheizt wird, und/oder die Druckkammer auf eine Druckkammertemperatur zwischen 10 K und 30 K oberhalb des Normalsiedepunkts der Probenlösung, insbesondere auf eine Druckkammertemperatur zwischen 110°C und 130°C geheizt wird.

## Claims

1. An arrangement having a microfluidic device (400) for the temperature-controlled processing of a sample solution and having a first and a second heating apparatus (452, 462), the microfluidic device (400) comprising a reaction chamber (450) in which the sample solution is heatable by means of the first heating apparatus (452) to a target temperature near the normal boiling point, the reaction chamber (450) being in fluid communication, via a compensating channel (422), with a pressure chamber (460) that is liquid-free in normal operation and that is heatable by means of the second heating apparatus (462) to a temperature above the normal boiling point of the sample solution, and that is designed to evaporate a quantity of sample liquid that is pushed by gas bubbles in the sample solution via the compensating channel (422) into the pressure chamber (460) and, in this way, to produce a gas-bubble-reducing counterpressure on the sample solution present in the reaction chamber (450), and the pressure chamber (460) comprising only a single opening and being in fluid communication with the reaction chamber (450) via said opening.

2. The arrangement according to claim 1, **characterized in that** the reaction chamber (450) is heatable by means of the first heating apparatus (452) to a target temperature between 10 K and 2 K below the normal boiling point of the sample solution, especially to a target temperature between 92°C and 98°C.

3. The arrangement according to one of claims 1 to 2, **characterized in that** the reaction chamber (450) is adapted for carrying out a PCR, especially a real-time PCR.

4. The arrangement according to one of claims 1 to 3, **characterized in that** the pressure chamber (460) is heatable by means of the second heating apparatus (462) to a pressure chamber temperature between 10 K and 30 K above the normal boiling point of the sample solution, especially to a pressure chamber temperature between 110°C and 130°C.

5. The arrangement according to one of claims 1 to 4, **characterized in that** the reaction chamber (450) is in fluid communication with an apparatus for detecting biomolecules in the sample solution.

6. The arrangement according to one of claims 1 to 5, **characterized in that** the microfluidic device is provided for a cell biological, analytical and/or diagnostic system, especially for a lab-on-a-chip system.

7. The arrangement according to one of claims 1 to 6, **characterized in that** the microfluidic device forms a closed system.

8. The arrangement according to one of claims 1 to 7, **characterized in that** the microfluidic device is developed in a microfluidic cartridge, especially a microfluidic cartridge consisting of polycarbonate.

9. The arrangement according to one of claims 1 to 8, **characterized in that** the first and second heating apparatus (452, 462) form external apparatuses with respect to the microfluidic device (400).

10. The arrangement according to one of claims 1 to 8, **characterized in that** the first and second heating apparatus (452, 462) are part of the microfluidic device (400).

11. A method for the temperature-controlled processing of a sample solution in a reaction chamber that is in fluid communication, via a compensating channel, with a pressure chamber that comprises only a single opening and is in fluid communication with the reaction chamber via said opening, in the method,
- the sample solution being supplied to the reaction chamber and heated to a target temperature near the normal boiling point of the sample solution, and
- the pressure chamber being heated to a temperature above the normal boiling point of the sample solution,
- such that the pressure chamber is liquid-free in normal operation and a quantity of sample liquid pushed by gas bubbles in the sample solution via the compensating channel into the pressure chamber evaporates in the pressure chamber and, in this way, produces a gas-bubble-reducing counterpressure on the sample solution present in the reaction chamber.

12. The method according to claim 11, **characterized in that** the reaction chamber is heated to a target temperature between 10 K and 2 K below the normal boiling point of the sample solution, especially to a target temperature between 92°C and 98°C, and/or the pressure chamber is heated to a pressure chamber temperature between 10 K and 30 K above the normal boiling point of the sample solution, especially to a pressure chamber temperature between 110°C and 130°C.

## Revendications

1. Système comportant un dispositif microfluidique (400) pour le traitement à température contrôlée d'une solution d'échantillon et un premier et un second dispositifs de chauffage (472, 462), le dispositif microfluidique (400) présentant une chambre de réaction (450) dans laquelle la solution d'échantillon peut être chauffée au moyen du premier dispositif de chauffage (452) à une température théorique proche du point d'ébullition normal, la chambre de réaction (450) étant en liaison fluidique par un canal de compensation (422) avec une chambre de compression (460) dépourvue de liquide en fonctionnement normal et qui peut être chauffée au moyen du second dispositif de chauffage (462) à une température supérieure au point d'ébullition normal de la solution d'échantillon et qui est conçue pour vaporiser une quantité de liquide d'échantillon comprimée par des bulles de gaz dans la solution d'échantillon par l'intermédiaire du canal de compensation (422) dans la chambre de compression (460) et pour générer ainsi une contre-pression réduisant les bulles de gaz sur la solution d'échantillon présente dans la chambre de réaction (450), et la chambre de compression (460) présentant seulement une ouverture et étant en liaison fluidique par cette ouverture avec la chambre de réaction (450).

2. Dispositif selon la revendication 1, **caractérisé en ce que** la chambre de réaction (450) peut être chauffée au moyen du premier dispositif de chauffage (452) à une température théorique de 10 K à 2 K en-dessous du point d'ébullition normal de la solution d'échantillon, en particulier à une température théorique entre 92 °C et 98 °C.

3. Dispositif selon une des revendications 1 à 2, **caractérisé en ce que** la chambre de réaction (450) est conçue pour réaliser une PCR, en particulier une PCR en temps réel.

4. Dispositif selon une des revendications 1 à 3, **caractérisé en ce que** la chambre de compression (460) peut être chauffée au moyen du second dispositif de chauffage (462) à une température de chambre de compression de 10 K à 30 K au-dessus du point d'ébullition normal de la solution d'échantillon, en particulier à une température de chambre de compression entre 110 °C et 130 °C.

5. Dispositif selon une des revendications 1 à 4, **caractérisé en ce que** la chambre de réaction (450) est en liaison fluidique avec un dispositif de mise en évidence de molécules biologiques dans la solution d'échantillon.

6. Dispositif selon une des revendications 1 à 5, **caractérisé en ce que** le dispositif microfluidique est prévu pour un système de biologie cellulaire, analytique et/ou de diagnostic, en particulier pour un système de laboratoire sur puce.

7. Dispositif selon une des revendications 1 à 6, **caractérisé en ce que** le dispositif microfluidique constitue un système fermé.

8. Dispositif selon une des revendications 1 à 7, **caractérisé en ce que** le dispositif microfluidique est réalisé dans une cartouche microfluidique, en particulier une cartouche microfluidique composée de polycarbonate.

9. Dispositif selon une des revendications 1 à 8, **caractérisé en ce que** les premier et second dispositifs de chauffage (452, 462) constituent des dispositifs externes par rapport au dispositif microfluidique (400).

10. Dispositif selon une des revendications 1 à 8, **caractérisé en ce que** les premier et second dispositifs de chauffage (452, 462) font partie intégrante du dispositif microfluidique (400).

11. Procédé de traitement à température contrôlée d'une solution d'échantillon dans une chambre de réaction qui est en liaison fluidique par un canal de compensation avec une chambre de compression qui présente seulement une ouverture et est en liaison fluidique par cette ouverture avec la chambre de réaction, sachant que, dans ce procédé,
- la solution d'échantillon est acheminée à la chambre de réaction et chauffée à une température théorique proche du point d'ébullition normale de la solution d'échantillon, et
- la chambre de compression est chauffée à une température supérieure au point d'ébullition normal de la solution d'échantillon,
- de sorte que la chambre de compression, en fonctionnement normal, est dépourvu de liquide et vaporise dans la chambre de compression une quantité de liquide d'échantillon comprimée par des bulles de gaz dans la solution d'échantillon par l'intermédiaire du canal de compensation dans la chambre de compression et génère ainsi une contre-pression réduisant les bulles de gaz sur la solution d'échantillon présente dans la chambre de réaction.

12. Procédé selon la revendication 11, **caractérisé en ce que** la chambre de réaction peut être chauffée à une température théorique entre 10 K et 2 K en-dessous du point d'ébullition normal de la solution d'échantillon, en particulier à une température théorique entre 92 °C et 98 °C, et/ou que la chambre de compression est chauffée à une température de chambre de compression entre 10 K et 30 K au-dessus du point d'ébullition normal de la solution d'échantillon, en particulier à une température de chambre de compression entre 110 °C et 130 °C.
